# EUROPEAN PATENT SPECIFICATION

(11) **EP 3 939 499 B1**
(45) Date of publication and mention of the grant of the patent: **04.06.2025**
(21) Application number: 21185823.8
(22) Date of filing: 15.07.2021
(51) Int. Cl.: A61B 5/0215, A61B 5/024, A61B 5/083, A61B 5/113, A61B 5/1455, A61B 5/00

(54) **PROCESSING DEVICE AND COMPUTER PROGRAM FOR DETERMINING RELIABILITY OF PHYSIOLOGICAL PARAMETER CALCULATION**
VERARBEITUNGSVORRICHTUNG UND COMPUTERPROGRAMM ZUR BESTIMMUNG DER VERLÄSSLICHKEIT VON PHYSIOLOGISCHER PARAMETERBERECHNUNG
DISPOSITIF DE TRAITEMENT ET PROGRAMME INFORMATIQUE PERMETTANT DE DETERMINER LA FIABILITÉ DU CALCUL DE PARAMÈTRES PHYSIOLOGIQUES

(30) Priority: 17.07.2020 JP 2020122965; 07.07.2021 JP 2021112727
(43) Date of publication of application: 19.01.2022
(73) Proprietor: Nihon Kohden Corporation, Shinjuku-ku Tokyo (JP)
(72) Inventor: Kawashima, Takuya, Tokorozawa-shi, Saitama (JP); Sano, Hiroto, Tokorozawa-shi, Saitama (JP); Matsuzawa, Wataru, Tokorozawa-shi, Saitama (JP)
(74) Representative: Grünecker Patent- und Rechtsanwälte PartG mbB

(56) References cited:
- WO-A1-2012/125135
- US-A1- 2015 150 485
- US-A1- 2017 252 513
- US-A1- 2019 133 468
- US-A1- 2020 029 911
- US-A1- 2020 205 704

## Description

### TECHNICAL FIELD

The presently disclosed subject matter relates to a physiological information acquisition device that acquires physiological information of a subject through a sensor. The presently disclosed subject matter also relates to a processing device that processes the physiological information, and a computer program.

### BACKGROUND ART

A physiological information acquisition device disclosed in JP-A-2018-102671 receives waveform data corresponding to a measurement waveform of a physiological parameter of a subject, and outputs an alarm indicating abnormality of at least one of a value of the physiological parameter and the measurement waveform thereof based on the waveform data. A further prior art device is known from US 2020/029911.

### SUMMARY OF INVENTION

An object of the presently disclosed subject matter is to detect a physiological parameter acquired in an acquisition environment low in reliability.

A device in accordance with the invention is defined in claim 1. Further, there is provided a processing device configured to process physiological information of a subject, and that can include:
a reception interface configured to receive first data corresponding to a measurement waveform of a first physiological parameter of the subject, and second data related to an acquisition environment of the first physiological parameter;
a predictor configured to predict a first probability of correctly calculating a value of the first physiological parameter, based on the first data; and
a processor configured to identify, of the first data, a part in which the first probability is higher than a first threshold under a circumstance where the first physiological parameter is determined to be abnormally acquired based on the second data.

A computer program in accordance with the invention is defined in claim 6. Further, there is provided a computer program that can be executed by a processor of a processing device configured to process physiological information of a subject, wherein: the computer program is executed to make the processing device perform the steps of:
receiving first data corresponding to a measurement waveform of a first physiological parameter of the subject, and second data related to an acquisition environment of the first physiological parameter;
predicting a first probability of correctly calculating a value of the first physiological parameter, based on the first data; and
identifying, of the first data, a part in which the first probability is higher than a first threshold under a circumstance where the first physiological parameter is determined to be abnormally acquired based on the second data.

Further, there is provided a physiological information acquisition device that can include:
a reception interface configured to receive first data corresponding to a measurement waveform of a first physiological parameter of the subject, and second data related to an acquisition environment of the first physiological parameter;
a predictor configured to predict a first probability of correctly calculating a value of the first physiological parameter, based on the first data;
a processor configured to identify, of the first data, a part in which the first probability is higher than a first threshold under a circumstance where the first physiological parameter is determined to be abnormally acquired based on the second data; and
a notifier configured to send notification about information indicating that the first physiological parameter is abnormally acquired when the first data part identified by the processor is present.

When the measurement waveform of the first physiological parameter is affected by noise, the first probability of correctly calculating the first physiological parameter may be determined to be higher than the first threshold though the first physiological parameter is actually abnormally acquired. In such a case, whether the first probability is correctly predicted or not can be verified by referring to the result of the determination as to whether the first physiological parameter is normally acquired or not based on the data related to the acquisition environment of the first physiological parameter. According to the configuration according to each of the aforementioned aspects, in the case where the first physiological parameter is determined to be abnormally acquired in spite of the first probability higher than the first threshold, the first data related to the first probability is concluded to be abnormally acquired. Accordingly, it is possible to detect the physiological parameter acquired in the acquisition environment low in reliability.

### BRIEF DESCRIPTION OF DRAWINGS

FIG. 1 is a block diagram illustrating a functional configuration of a physiological information acquisition device according to an embodiment.
FIG. 2 is a flow chart for explaining a method for generating a predictor in FIG. 1.
FIG. 3 is a graph illustrating the relationship between electrocardiogram waveform data and first heart rate data.
FIG. 4 is a graph illustrating the relationship between the electrocardiogram waveform data and second heart rate data.
FIG. 5 is a graph illustrating data indicating a change over time in heart rate acquired from a subject.
FIG. 6 is a graph illustrating data indicating a change over time in heart rate processed by the predictor.
FIG. 7 is a flow chart for explaining an example of processing performed by a processor in FIG. 1.
FIG. 8 is Graphs for explaining the processing of FIG. 7.
FIG. 9 is Graphs for explaining the processing of FIG. 7.
FIG. 10 is a flow chart for explaining another example of the processing performed by the processor in FIG. 1.
FIG. 11 is Graphs for explaining the processing of FIG. 10.
FIG. 12 is a flow chart for explaining a further example of the processing performed by the processor in FIG. 1.

### DESCRIPTION OF EMBODIMENT

An embodiment will be described in detail below by way of example with reference to the accompanying drawings.

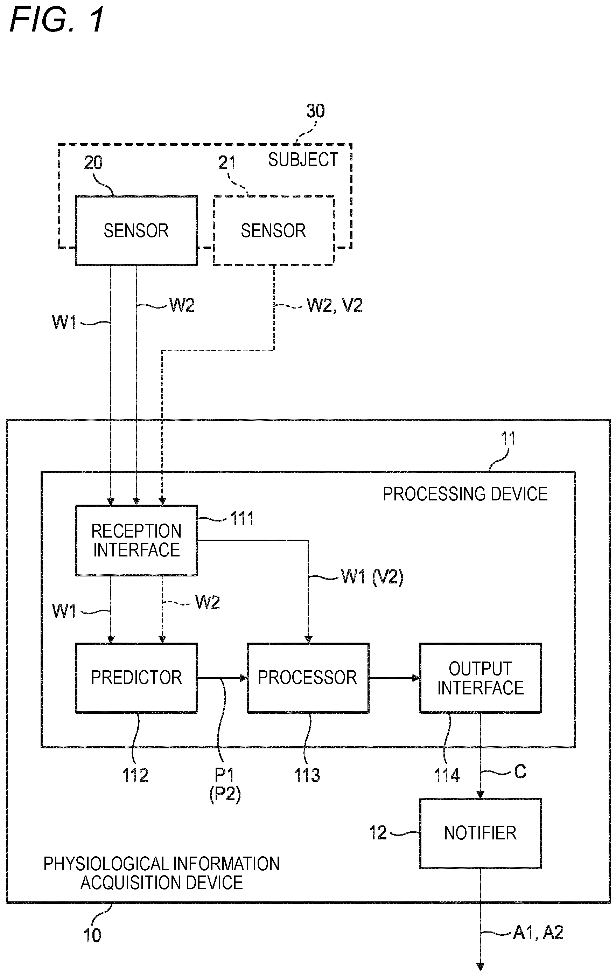

FIG. 1 illustrates a functional configuration of a physiological information acquisition device 10 according to an embodiment. The physiological information acquisition device 10 is an apparatus that acquires physiological parameters of a subject 30 through a sensor 20. Examples of the sensor 20 include an electrode for acquiring a heart rate, an electrode for acquiring an impedance respiration rate, a catheter for acquiring a value of invasive blood pressure, a probe for acquiring a percutaneous arterial oxygen saturation (SpO2) or a pulse rate, a sensor for acquiring a carbon dioxide concentration of respiratory air, etc.

The physiological information acquisition device 10 includes a processing device 11. The processing device 11 includes a reception interface 111, a predictor 112, a processor 113, and an output interface 114.

The reception interface 111 is configured so as to receive first waveform data W1 corresponding to a measurement waveform of a first physiological parameter of the subject 30, and second waveform data W2 corresponding to a measurement waveform of a second physiological parameter of the subject 30 from the sensor 20. Each of the first waveform data W1 and the second waveform data W2 may have an analog data form or may have a digital data form. In the case where the first waveform data W1 and the second waveform data W2 have the analog data form, the reception interface 111 is configured so as to include a suitable conversion circuit including an A/D converter. The predictor 112 and the processor 113 process the first waveform data W1 and the second waveform data W2 which have a digital data form. The first waveform data W1 is an example of first data. The second waveform data W2 is an example of second data.

The predictor 112 is configured so as to predict a first probability P1 of correctly calculating a value of the first physiological parameter, based on the first waveform data W1. The predictor 112 is realized as a trained model or a discriminator generated by machine learning that will be described later.

The processor 113 is configured so as to determine whether the second physiological parameter is normally acquired or not, based on the second waveform data W2. Specifically, in a case where a value of the second physiological parameter that can be acquired from the second waveform data W2 is out of a threshold range, or a case where a feature amount of the second waveform data W2 is out of a threshold range, the processor 113 determines that the second physiological parameter is abnormally acquired. The feature amount of the measurement waveform is appropriately decided according to the type of the acquired second physiological parameter. Since the determination technique itself has been well known, detailed description thereof will be omitted.

The processor 113 can be realized by a general-purpose microprocessor that operates in cooperation with a general-purpose memory. Examples of the general-purpose microprocessor may include a CPU, an MPU, and a GPU. Examples of the general-purpose memory may include an ROM and an RAM. In this case, a computer program executing processing that will be described later can be stored in the ROM. The ROM is an example of a storage medium storing the computer program. The processor designates at least a part of the computer program stored on the ROM, expands the designated part of the computer program on the RAM, and performs the aforementioned processing in cooperation with the RAM. The aforementioned computer program may be pre-installed in the general-purpose memory, or may be downloaded from an external server device through a communication network and installed in the general-purpose memory. In this case, the external server device is another example of the storage medium storing the computer program.

The processor 113 may be realized by a dedicated integrated circuit, such as a microcontroller, an ASIC or an FPGA, capable of executing the aforementioned computer program. In this case, the aforementioned computer program is pre-installed in a storage element included in the dedicated integrated circuit. The storage element is a further example of the storage medium storing the computer program. The processor 113 can also be realized by a combination of the general-purpose microprocessor and the dedicated integrated circuit.

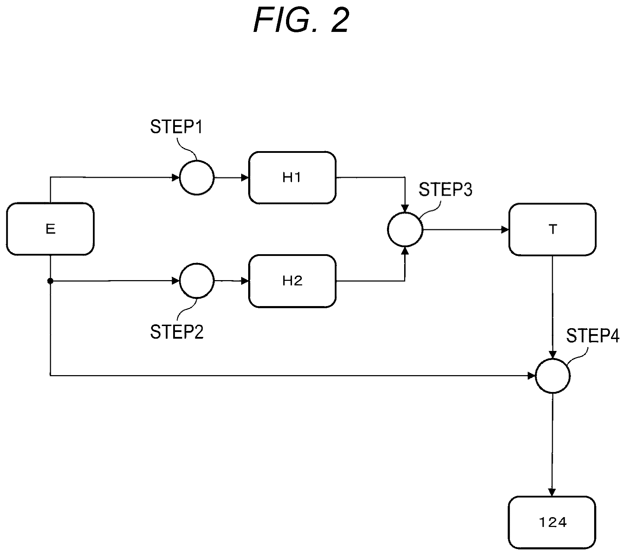

A method for generating the predictor 112 capable of predicting a probability of erroneously calculating a heart rate based on a measurement waveform of an electrocardiogram will be described by way of example with reference to FIG. 2.

First, a process of acquiring first heart rate data H1 from electrocardiogram waveform data E is performed (STEP 1). The first heart rate data H1 is data indicating a value of a heart rate correctly calculated from the electrocardiogram waveform data E.

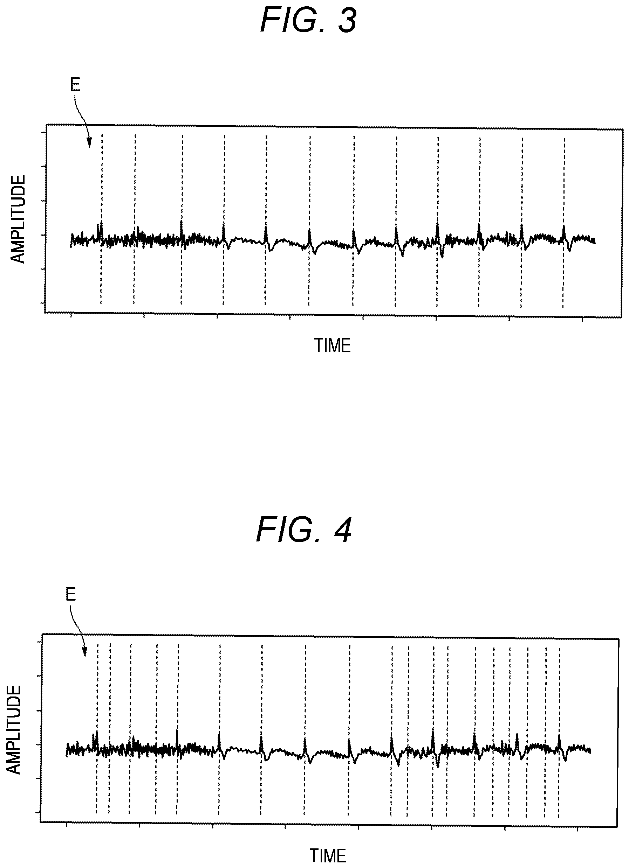

FIG. 3 illustrates the relationship between the electrocardiogram waveform data E and the first heart rate data H1. The electrocardiogram waveform data E indicates a change over time in physiological electrical activity of a heart of a subject. The electrocardiogram waveform data E changes with beating of the heart. The first heart rate data H1 is calculated by counting the number of times of such a change per minute. In FIG. 3, each broken line extending in an up/down direction represents a portion used for counting the heart rate in the electrocardiogram waveform data E. In FIG. 3, twelve heartbeats are counted within a time (e.g. ten seconds) in which the waveform is displayed.

The heart rate corresponding to the first heart rate data H1 may be counted by visually observing the electrocardiogram waveform data E, or may be counted using appropriate counting software. As the electrocardiogram waveform data E, waveform data actually acquired from the subject using an electrocardiogram sensor or the like may be used, or waveform data laid open as a database for performance evaluation of an electrocardiogram monitor may be used.

Subsequently to or in parallel with the STEP 1, a process (STEP 2 in FIG. 2) of acquiring second heart rate data H2 from the electrocardiogram waveform data E is performed. The second heart rate data H2 is data indicating a value of the heart rate automatically calculated from the electrocardiogram waveform data E by a specific heart rate calculation algorithm. The heart rate calculation algorithm can be executed, for example, by the processor 113 of the processing device 11 mounted on the physiological information acquisition device 10. The heart rate calculation algorithm is configured so as to automatically calculate the heart rate based on features or the like of a waveform shape in the inputted electrocardiogram waveform data E.

FIG. 4 illustrates the relationship between the electrocardiogram waveform data E and the second heart rate data H2. The electrocardiogram waveform data E illustrated in FIG. 4 is the same as the electrocardiogram waveform data E illustrated in FIG. 3. Based on the same electrocardiogram waveform data E, nineteen heartbeats rather than twelve heartbeats are counted in the example shown in FIG. 4. This fact means that there is a possibility that the heart rate calculation algorithm used for acquisition of the second heart rate data H2 performs counting based on erroneously detected heartbeats.

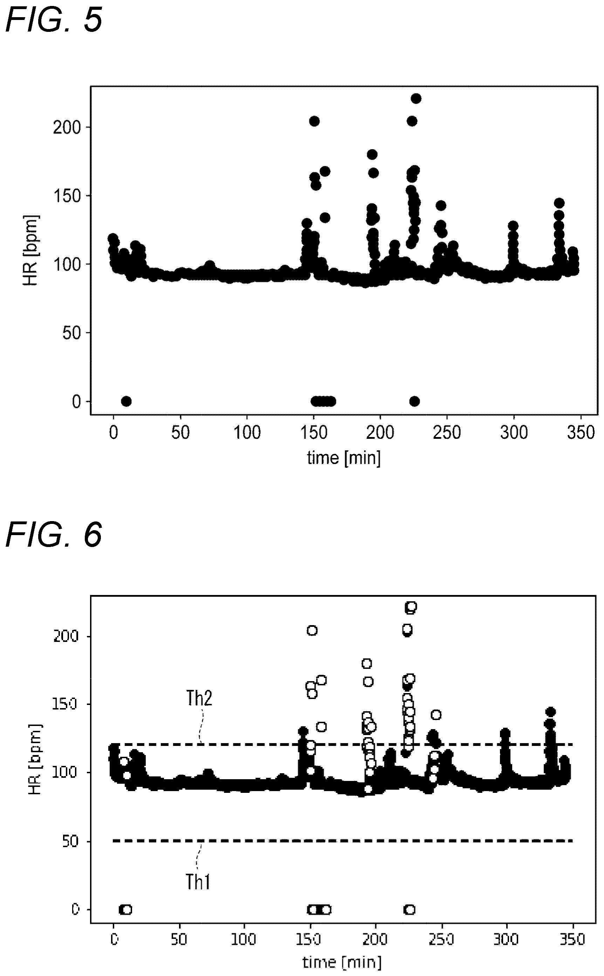

FIG. 5 illustrates a change over time in the heart rate automatically calculated by the aforementioned heart rate calculation algorithm to which the electrocardiogram waveform data E actually acquired from the subject has been inputted. Each of points included in the graph of FIG. 5 corresponds to a heart rate detected within a predetermined time (e.g. one minute).

Of values of the heart rate illustrated in FIG. 5, erroneously calculated values are represented by white circles in FIG. 6. One technique that can exclude such erroneously calculated heart rate data is to set a threshold range for the heart rate. For example, all heart rate data parts where the heart rate does not fall between a lower threshold Th1 and an upper threshold Th2 may be excluded. However, as illustrated in FIG. 6, some heart rate data parts where the heat rate was erroneously calculated may fall into the threshold range, and conversely, other heart rate data parts where the heat rate was correctly calculated may fall out of the threshold range. The former is based on misanalysis of the heart rate calculation algorithm, and the latter corresponds to a case where the heart rate increases due to some physiological phenomenon in the subject. The misanalysis of the heart rate calculation algorithm can be caused by, for example, a phenomenon in which a T wave high in amplitude is erroneously recognized as a QRS complex and reduplicatively counted as a heartbeat (so-called double count). The misanalysis of the heart rate calculation algorithm can also be caused by body motion of the subject or external noise such as deterioration or drop-off of the electrode attached to the subject.

Subsequently, performed is a process (STEP 3 in FIG. 2) of collating the second heart rate data H2 corresponding to the heart rate automatically calculated by the heart rate calculation algorithm with the first heart rate data H1 so as to generate teacher data T including a teacher label indicating whether the heart rate is correct or incorrect.

In the case where the first heart rate data H1 illustrated in FIG. 3 and the second heart rate data H2 illustrated in FIG. 4 are used to be collated with each other, the teacher data T includes a teacher label indicating that the heart rate corresponding to the second heart rate data H2 is incorrect.

In addition, the heart rate corresponding to the first heart rate data H1 and the heart rate corresponding to the second heart rate data H2 do not have to be always completely identical with each other in order to include a teacher label indicating that the heart rate corresponding to the second heart rate data H2 is correct. If an error of the heart rate corresponding to the second heart rate data H2 with respect to the heart rate corresponding to the first heart rate data H1 is within an allowable range, the teacher data T may include the teacher label indicating the heart rate corresponding to the second heart rate data H2 is correct.

That is, the heart rate automatically calculated by the heart rate calculation algorithm is used to be collated with the heart rate known to have been calculated correctly. Therefore, the teacher label obtained as a result of the collation can reflect a tendency or habit that the heart rate calculation algorithm correctly or incorrectly calculates the heart rate with respect to the inputted electrocardiogram waveform data E.

Subsequently, machine learning of the predictor 112 is performed based on the combination of the electrocardiogram waveform data E and the teacher data T (STEP 4 in FIG. 2). Any well-known technique related to supervised learning can be appropriately used as the machine learning. That is, the predictor 112 learns a feature amount of the electrocardiogram waveform data E in which the heart rate was erroneously calculated, and an arithmetic processing parameter is modified so that the predictor 112 can output prediction data indicating that a probability of correctly calculating the heart rate is low when the electrocardiogram waveform data E having such a feature amount is inputted. The arithmetic processing parameter may be modified by a learning executor or by the predictor 112 itself through the learning. The prediction data may indicate the calculated probability itself, or may be associated with a score (for example, any value selected from 1 to 5) or the like corresponding to the probability.

In the aforementioned example, the first heart rate data H1 and the second heart rate data H2 are acquired using the shared electrocardiogram waveform data E in order to generate the predictor 112 for predicting the probability of erroneously calculating the heart rate. As described above, the first heart rate data H1 is data indicating the heart rate that was correctly calculated from the electrocardiogram waveform data E. The second heart rate data H2 is data indicating the heart rate that was calculated by the specific heart rate calculation algorithm. There is a possibility that the second heart rate data H2 includes an erroneously calculated heart rate.

As another example, first respiration rate data and second respiration rate data may be acquired using shared capnogram data in order to generate the predictor 112 for predicting a probability of erroneously calculating a respiration rate. The capnogram data is an example of the waveform data. The respiration rate is an example of the physiological parameter. The first respiration rate data is data indicating a respiration rate that was correctly calculated from the capnogram data. The second respiration rate data is data indicating a respiration rate that was calculated by a specific respiration rate calculation algorithm. There is a possibility that the second respiration rate data includes an erroneously calculated respiration rate.

A physiological parameter that is used as a target for estimating an erroneous calculation probability by the predictor 112 may be different from a physiological parameter that is used as a target for outputting an alarm. For example, the predictor 112 for predicting a probability of erroneously calculating a heart rate can be used for a configuration in which an alarm is outputted for abnormality of an ST value, a QT interval, QTc, or the like in the electrocardiogram waveform data. Alternatively, the predictor 112 for predicting a probability of erroneously calculating a respiration rate can be used for a configuration in which an alarm is outputted for abnormality of an end-tidal carbon dioxide (EtCO2) or the like in the capnogram data.

Different types of physiological parameters may be used for generation of the predictor 112. As an example, heart rate data acquired from electrocardiogram waveform data and pulse rate data acquired from invasive arterial blood pressure waveform data can be used in order to generate the predictor 112 for predicting a probability of erroneously calculating a pulse rate. The electrocardiogram waveform data and the invasive arterial blood pressure waveform data need to be acquired from one and the same subject. The heart rate data is data indicating a heart rate that was correctly calculated from the electrocardiogram waveform data. The pulse rate data is data indicating a pulse rate that was calculated by a specific pulse rate calculation algorithm. There is a possibility that the pulse rate data includes an erroneously calculated pulse rate.

The predictor 112 generated in the aforementioned manner can be used for predicting a probability of erroneously calculating invasive arterial blood pressure.

Pulse waveform data may be used in order to obtain the pulse rate data. The pulse waveform data can be acquired by a pulse photometry probe or the like that non-invasively measures a change over time in absorbance of blood. Alternatively, the pulse waveform data can be acquired by a non-invasive arterial blood pressure gauge or the like that measures a change over time in internal pressure of a cuff. In this case, the generated predictor 112 is used for predicting a probability of erroneously calculating non-invasive arterial blood pressure, a pulse rate, or a percutaneous arterial oxygen saturation (SpO2).

As another example, first respiration rate data acquired from impedance respiration waveform data and second respiration rate data acquired from capnogram data can be used in order to generate the predictor 112 for predicting a probability of erroneously calculating a respiration rate. The impedance respiration waveform data and the capnogram data need to be acquired from one and the same subject. The first respiration rate data is data indicating a respiration rate that was correctly calculated from the impedance respiration waveform data. The second respiration rate data is data indicating a respiration rate that was calculated by the specific respiration rate calculation algorithm. There is a possibility that the second respiration rate data includes an erroneously calculated respiration rate.

The processor 113 is configured so as to identify a part of the first waveform data W1 where the first probability P1 predicted by the predictor 112 is higher than a first threshold P1t under a circumstance where the second physiological parameter is determined to be abnormally monitored.

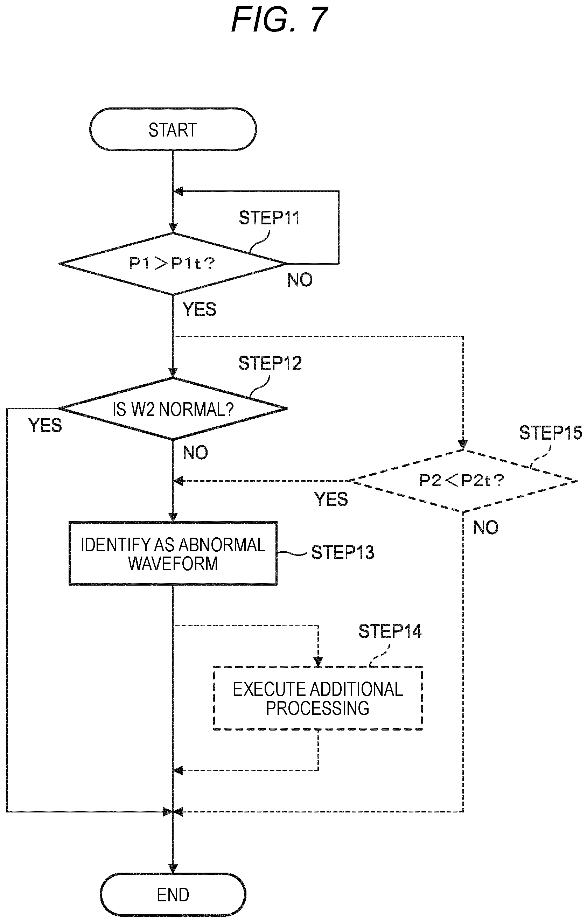

A processing flow executed by the processor 113 configured thus will be described by way of example with reference to FIG. 7. In accordance with the invention, the first waveform data W1 corresponds to a measurement waveform of impedance respiration. As the first physiological parameter, a respiration rate of the subject 30 is acquired. The second waveform data W2 corresponds to a measurement waveform of an electrocardiogram. As the second physiological parameter, a heart rate of the subject 30 is acquired. Therefore, the predictor 112 generates teacher data T and performs machine learning so as to output prediction data corresponding to a first probability P1 of correctly calculating the respiration rate based on the first waveform data W1 corresponding to the measurement waveform of the inputted impedance respiration.

Incidentally, the first waveform data W1 corresponding to the measurement waveform of the impedance respiration and the second waveform data W2 corresponding to the measurement waveform of the electrocardiogram can be acquired from the shared sensor 20 (electrode).

The processor 113 determines whether the first waveform data W1 in which the first probability P1 of correctly calculating the respiration rate is higher than a first threshold P1t is present or not (STEP 11). The processing is repeated until the first waveform data W1 in which the first probability P1 is higher than the first threshold P1t is identified (NO in STEP 11).

When the first waveform data W1 in which the first probability P1 is higher than the first threshold P1t has been identified (YES in STEP 11), the processor 113 determines whether the second physiological parameter is normally acquired or not (STEP 12).

When determination has been made that the second physiological parameter is normally acquired (YES in STEP 12), the processor 113 ends the processing.

When determination has been made that the second physiological parameter is abnormally acquired (NO in STEP 12), the processor 113 identifies a part of the first waveform data W1 used for the determination, as a waveform data part corresponding to an abnormal measurement waveform (STEP 13).

When the measurement waveform of the respiration rate is affected by noise, there may arise a case where the first probability P1 of correctly calculating the respiration rate is higher than the first threshold P1t though the respiration rate is actually abnormally acquired. In the configuration according to the present invention, whether the first probability P1 is correctly predicted or not is verified by referring to the result of the determination as to whether the heart rate is normally acquired or not in such a case. Specifically, when the heart rate is determined to be abnormally acquired in spite of the first probability P1 higher than the first threshold Pt1, the first waveform data W1 related to the first probability P1 is concluded to be abnormally acquired. Therefore, the respiration rate acquired in an acquisition environment low in reliability can be detected.

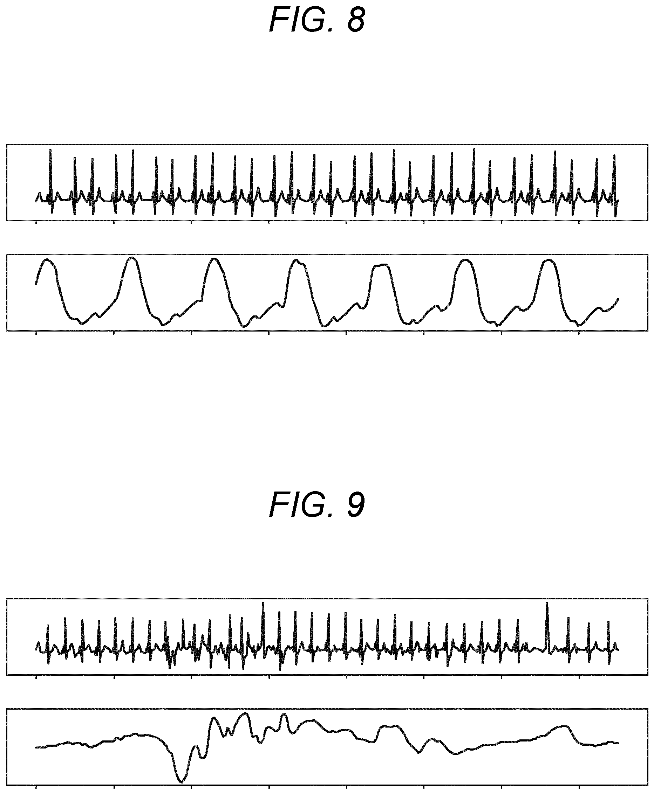

From a viewpoint of detecting the physiological parameter acquired in the acquisition environment low in reliability, it is preferable that the first physiological parameter and the second physiological parameter that have a high correlation to each other are appropriately selected. Two parameters that can be acquired through the shared sensor 20, such as the impedance respiration waveform and the electrocardiogram waveform in the present example, tend to have a high correlation to each other. FIG. 8 illustrates an electrocardiogram waveform and an impedance respiration waveform that are normally acquired. FIG. 9 illustrates an electrocardiogram waveform and an impedance respiration waveform that are affected by noise. In each of FIG. 8 and FIG. 9, the electrocardiogram waveform is illustrated on an upper side and the impedance respiration waveform is illustrated on a lower side. Therefore, the respiratory rate detected in the acquisition environment low in reliability can be detected by referring to whether the heart rate is normally acquired or not.

In particular, the impedance respiration waveform has convenience of being able to be obtained by use of the electrode shared with the electrocardiogram waveform but has a property of being easily affected by noise. However, since the impedance respiration waveform affected by the noise can be accurately identified by referring to the state of the electrocardiogram waveform highly correlated to the impedance respiration waveform, it is easy to enjoy the benefit of the convenience.

As illustrated in FIG. 1, the physiological information acquisition device 10 includes a notifier 12. The notifier 12 is configured to notify a user of the part of the first waveform data W1 that was identified as abnormal based on the aforementioned technique. Specifically, the processor 113 of the processing device 11 outputs control data C from the output interface 114 to control the notifier 12 to notify the user so that the user can recognize that the identified part of the first waveform data W1 is abnormal.

The control data C transmitted to the notifier 12 may have an analog data form, or may have a digital data form. When the control data C has the analog data form, the output interface 114 is configured to include an appropriate conversion circuit including a D/A converter.

The notification performed by the notifier 12 can include at least one of visible notification, audible notification, and tactile notification. The notification can be made so that among calculation results of the respiration rate used for display based on the first waveform data W1, a calculation result of the respiration rate calculated based on the part of the first waveform data W1 that was identified as abnormal can be displayed in another color, for example, in a manner similar to or the same as in the example illustrated in FIG. 6. According to such a configuration, it is possible to easily identify a case where the respiratory rate was abnormally acquired in spite of prediction that the respiratory rate would be highly likely to be calculated correctly, and it is also possible to provide information for analyzing a situation that the prediction was not made correctly.

As illustrated in FIG. 7, the processor 113 of the processing device 11 can execute additional processing on the part of the first waveform data W1 that was identified as abnormal (STEP 14).

As an example, the processor 113 can delete the part of the first waveform data W1 that was identified as abnormal. According to such a configuration, it is possible to reduce a burden of reorganizing the information provided by the user, and it is possible to also reduce a processing load related to the presentation of the part of the first waveform data W1 that was identified as abnormal.

As another example, the processor 113 can delete the first waveform data W1 except the part of the first waveform data W1 that was identified as abnormal. According to such a configuration, it is easy to grasp the tendency of the waveform data that is apt to be erroneously calculated by the current algorithm, so that it is possible to acquire information related to an improvement of the algorithm.

In addition to or in place of each of the aforementioned examples, the processor 113 can update the first probability P1 estimated in the part of the first waveform data W1 that was identified as abnormal. For example, the processor 113 can update the first probability P1 to a value not higher than the first threshold P1t. According to such a configuration, the first probability P1 is treated to take an appropriate value in the part of the first waveform data W1 that was identified as abnormal. There is an advantage in such a case that a subsequent process uses the first probability P1.

A process of determination as to whether the second physiological parameter is normally acquired or not can be performed by use of the predictor 112. In this case, the predictor 112 is configured so as to predict a second probability P2 of correctly calculating the value of the second physiological parameter based on the second waveform data W2. In the aforementioned example, the predictor 112 generates teacher data T and performs machine learning so as to output prediction data corresponding to the second probability P2 of correctly calculating the heart rate based on the second waveform data W2 corresponding to the measurement waveform of the inputted electrocardiogram.

Specifically, as illustrated in FIG. 7, the processor 113 determines whether the second probability P2 predicted by the predictor 112 is lower than a second threshold P2t or not (STEP 15). When the second probability P2 is lower than the second threshold P2t (YES in the STEP 15), the processor 113 determines that the second physiological parameter is abnormally acquired, and moves the processing forward to the STEP 13. When the second probability P2 is not lower than the second threshold value P2t (NO in the STEP15), the processor 113 ends the processing.

In the aforementioned example, the reception interface 111 of the processing device 11 receives the first waveform data W1 corresponding to the measurement waveform of the first physiological parameter and the second waveform data W2 corresponding to the measurement waveform of the second physiological parameter from the shared sensor 20. However, as illustrated in FIG. 1, the second waveform data W2 may be received from a sensor 21 different from the sensor 20. For example, the sensor 20 can be configured to acquire the electrocardiogram waveform data of the subject, and the sensor 21 can be configured to acquire the pulse waveform data of the subject. In this case, the heart rate is an example of the first physiological parameter, and the pulse rate is an example of the second physiological parameter. That is, the electrocardiogram waveform data is an example of first data, and the pulse waveform data is an example of second data.

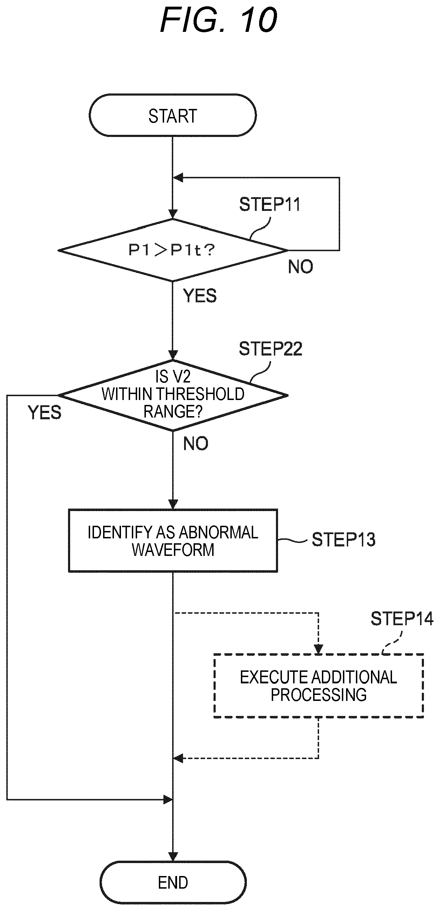

Alternatively, the reception interface 111 of the processing device 11 can receive measurement value data V2 corresponding to a measurement value of the second physiological parameter from the sensor 21. FIG. 10 explains an example of a processing flow that can be executed by the processor 113 in this case. Processes that are substantially the same as the processes described with reference to FIG. 7 will be designated by the same STEP numbers, and repeated description thereof will be omitted.

In the present unclaimed example, the first waveform data W1 corresponds to a measurement waveform of invasive arterial blood pressure. As the first physiological parameter, the invasive arterial blood pressure of the subject 30 is acquired. Therefore, the predictor 112 generates teacher data T and performs machine learning so as to output prediction data corresponding to a first probability P1 of correctly calculating the invasive arterial blood pressure, based on the first waveform data W1 corresponding to the measurement waveform of the inputted invasive arterial blood pressure.

In the present example, non-invasive arterial blood pressure of the subject 30 is acquired as the second physiological parameter. That is, the sensor 21 can be realized by a cuff attached to the subject 30. The measurement value data V2 corresponds to a measurement value of the non-invasive arterial blood pressure. The measurement value data V2 is an example of the second data.

The processor 113 determines whether the first waveform data W1 in which a first probability P1 of correctly calculating the invasive arterial blood pressure is higher than a first threshold P1t is present or not (STEP 11). The processing is repeated until the first waveform data W1 in which the first probability P1 is higher than the first threshold P1t is identified (NO in the STEP 11).

When the first waveform data W1 in which the first probability P1 is higher than the first threshold P1t has been identified (YES in the STEP 11), the processor 113 determines whether the non-invasive arterial blood pressure corresponding to the measurement value data V2 is within a threshold range or not (STEP 22).

When determination has been made that the non-invasive arterial blood pressure is within the threshold range (YES in the STEP 22), the processor 113 ends the processing.

When determination has been made that the second physiological parameter is abnormally acquired (NO in the STEP 22), the processor 113 identifies the first waveform data W1 used for the determination, as waveform data corresponding to an abnormal measurement waveform (STEP 13).

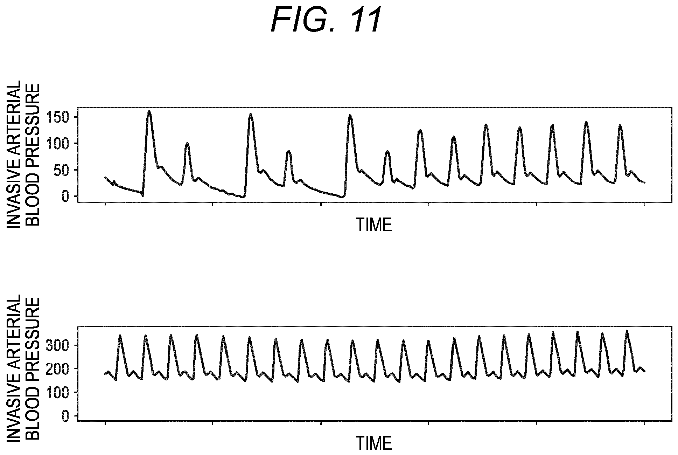

When an acquisition environment for the invasive arterial blood pressure is not appropriate, the invasive arterial blood pressure may show an abnormal measurement value while the measurement waveform itself is maintained as it is. When there is abnormality in zero calibration during acquisition of the invasive arterial blood pressure, the invasive arterial blood pressure can show an abnormal value while the measurement waveform itself of the invasive arterial blood pressure remains normal, for example, as illustrated in FIG. 11. In an example illustrated on an upper side of FIG. 11, a diastolic blood pressure value is abnormally low. In an example illustrated on a lower side of FIG 11, a systolic blood pressure value is abnormally high. However, since the measurement waveform itself is normal in each of these examples, there may arise a case where the first probability P1 of correctly calculating the invasive arterial blood pressure is determined to be higher than the first threshold P1t.

In the configuration according to the present example, whether the first probability P1 is correctly predicted or not can be verified by referring to a measurement value of the non-invasive arterial blood pressure in such a case. Specifically, when the measurement value of the non-invasive arterial blood pressure is out of a threshold range in spite of the first probability P1 higher than the first threshold Pt1, the first waveform data W1 related to the first probability P1 is concluded to be abnormally acquired. Therefore, the invasive arterial blood pressure acquired in an acquisition environment low in reliability can be detected.

In a manner similar to or the same as the example described with reference to FIG. 7, the processor 113 of the processing device 11 can output control data C from the output interface 114 to control the notifier 12 to notify the user so that the user can recognize that the identified first waveform data W1 is abnormal.

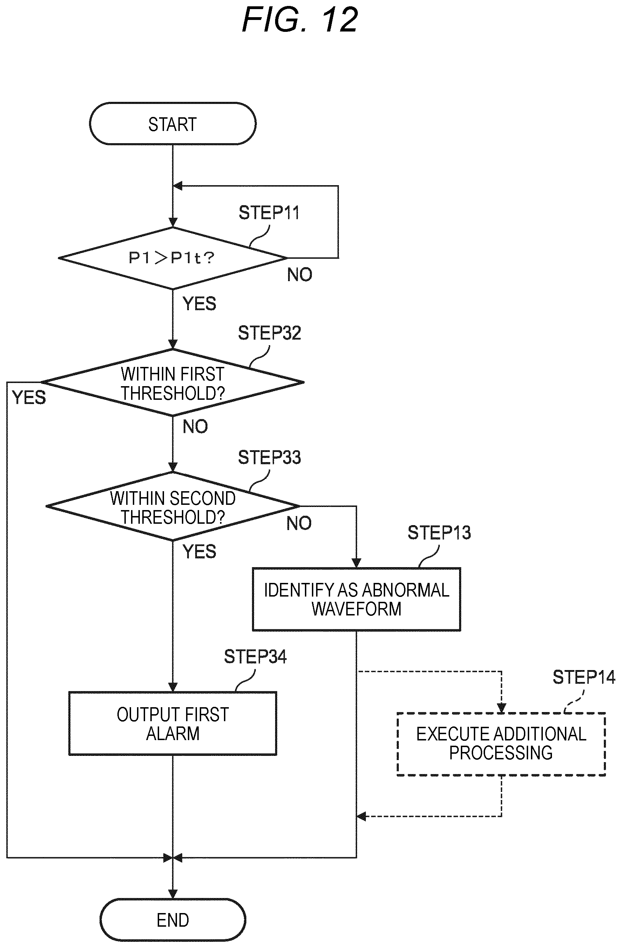

Next, an example of processing that can be executed by the processor 113 of the processing device 11 using only first waveform data W1 will be described with reference to FIG. 12. Processes that are substantially the same as the processes described with reference to FIG. 7 will be designated by the same STEP numbers, and repeated description thereof will be omitted.

In the present unclaimed example, the first waveform data W1 corresponds to a measurement waveform of invasive arterial blood pressure. As a first physiological parameter, invasive arterial blood pressure of a subject 30 is acquired. Therefore, the predictor 112 generates teacher data T and performs machine learning so as to output prediction data corresponding to a first probability P1 of correctly calculating the invasive arterial blood pressure based on the first waveform data W1 corresponding to the measurement waveform of the inputted invasive arterial blood pressure.

As illustrated in FIG. 1, the notifier 12 according to the present example is configured to be able to output a first alarm A1 and a second alarm A2. The first alarm A1 is outputted in order to indicate that the invasive arterial blood pressure is out of a first threshold range. The first threshold range can be, for example, set as a range of from 70 mmHg to 180 mmHg. The first threshold range is decided as a range for determining that the invasive arterial blood pressure of the subject 30 is abnormal. The second alarm A2 can be outputted in order to indicate abnormality of an acquisition state of the invasive arterial blood pressure. Each of the first alarm A1 and the second alarm A2 can include at least one of a visible alarm, an audible alarm, and a tactile alarm.

The processor 113 determines whether the first waveform data W1 in which a first probability P1 of correctly calculating the invasive arterial blood pressure is higher than a first threshold value P1t is present or not (STEP 11). The processing is repeated until the first waveform data W1 in which the first probability P1 is higher than the first threshold value P1t is identified (NO in the STEP 11).

When the first waveform data W1 in which the first probability P1 is higher than the first threshold value P1t is identified (YES in the STEP 11), the processor 113 determines whether a measurement value of the invasive arterial blood pressure identified from the first waveform data W1 is within the first threshold range or not (STEP 32). The measurement value of the invasive arterial blood pressure is an example of second data.

When determination has been made that the invasive arterial blood pressure is within the first threshold range (YES in the STEP 32), the processor 113 ends the processing.

When determination has been made that the invasive arterial blood pressure is out of the first threshold range (NO in the STEP 32), the processor 113 determines whether the invasive arterial blood pressure calculated from the first waveform data W1 is within a second threshold range or not (STEP 33). The second threshold range is decided to be wider than the first threshold range and to include the first threshold range. The second threshold range can be, for example, set as a range of from 20 mmHg to 300 mmHg. Any value that is out of the second threshold range is a value that cannot be normally taken by invasive arterial blood of the subject 30, but it is a value that can be taken when zero calibration peculiar to measurement of the invasive arterial blood pressure was abnormally performed.

When determination has been made that the invasive arterial blood pressure is out of the first threshold range but within the second threshold range (YES in STEP 33), the processor 113 outputs control data C from the output interface 114 to control the notifier 12 to output the first alarm A1 (STEP 34). As a result, the user is notified of the fact that the invasive arterial blood pressure of the subject 30 is abnormal.

When determination has been made that the invasive arterial blood pressure is out of the second threshold range (NO in STEP 33), the processor 113 identifies a part of the first waveform data W1 used for the determination, as a waveform data part corresponding to an abnormal acquisition state (STEP 13).

When the acquisition state of the invasive arterial blood pressure is abnormal as described with reference to FIG. 11, the invasive arterial blood pressure may show an abnormal measurement value while the measurement waveform itself is maintained as it is. However, when the measurement waveform itself is normal, there may arise a case where it is determined that the first probability P1 of correctly calculating the invasive arterial blood pressure is higher than the first threshold P1t.

In the configuration according to the present example, whether the first probability P1 is correctly predicted or not can be verified by referring to the measurement value of the invasive arterial blood pressure in such a case. Specifically, the second threshold range is set to include the first threshold range that can be taken by the invasive arterial blood pressure when the subject is normal, and to be wider than the first threshold range. When the invasive arterial blood pressure is determined to be out of the second threshold range in spite of the first probability P1 higher than the first threshold Pt1, conclusion is made that the part of the first waveform data W1 related to the first probability P1 was acquired under an abnormal state. Therefore, the invasive arterial blood pressure which was acquired in the acquisition environment low in reliability can be detected.

The processor 113 can output control data C from the output interface 114 to control the notifier 12 to output at least the second alarm A2 when the part of the first waveform data W1 identified as having been acquired under the abnormal state is present. As a result, the user is notified of the abnormality of the acquisition state of the invasive arterial blood pressure. Since the invasive arterial blood pressure is out of the first threshold range, the output of the first alarm A1 may be also performed under the situation where the second alarm A2 is outputted.

In addition to or in place of the output of the second alarm A2, notification can be made in a manner similar to or the same as the example of FIG. 6 so that, of calculation results of the invasive arterial blood pressure used for display based on the first waveform data W1, a calculation result of the invasive arterial blood pressure calculated based on the part of the first waveform data W1 identified as abnormal is displayed in another color. According to such a configuration, it is possible to easily identify the case where the invasive arterial blood pressure is abnormally acquired in spite of prediction that the invasive arterial blood pressure would be highly likely to be calculated correctly, and it is also possible to provide information for analyzing a situation in which the prediction was made incorrectly.

As illustrated in FIG. 12, the processor 113 of the processing device 11 can execute additional processing on the part of the first waveform data W1 identified as abnormal (STEP 14).

In a manner similar to or the same as the example described with reference to FIG. 10, the reception interface 111 of the processing device 11 can receive measurement value data V2 corresponding to a measurement value of non-invasive arterial blood pressure of the subject 30 from the sensor 21. In this case, the processor 113 of the processing device 11 can adjust at least one of the first threshold range and the second threshold range based on the measurement value of the non-invasive arterial blood pressure corresponding to the measurement value data V2 as an example of the additional processing. Specifically, based on the calculated non-invasive arterial blood pressure, a normal value range of the invasive arterial blood pressure of the subject 30 that can be assumed under a normal zero calibration condition is decided, and at least one of the first threshold range and the second threshold range is set again.

According to such a configuration, an appropriate numerical value range is decided for at least one of the first threshold range and the second threshold range. Accordingly, it is possible to increase detection accuracy of the invasive arterial blood pressure acquired in the acquisition environment low in reliability.

The aforementioned embodiment is merely illustrated in order to make it easy to understand the presently disclosed subject matter. The configuration according to the aforementioned embodiment can be appropriately modified or improved without departing from the spirit of the presently disclosed subject matter.

In the aforementioned embodiment, the data referred to during the verification of the first probability P1 of correctly calculating the value of the first physiological parameter is the measurement waveform or the measurement value of the physiological parameter of the subject 30. However, the data can be acceleration data outputted from an acceleration sensor mounted on the subject 30, or image data obtained by photographing the subject 30. In this case, the acceleration data or the image data is an unclaimed example of the second data.

Both the acceleration data and the image data can correspond to the body motion of the subject 30. For example, when determination has been made that the body motion of the subject 30 is large from the data in spite of the first probability P1 higher than the first threshold Pt1, the processor 113 of the processing device 11 concludes that the first waveform data W1 related to the first probability P1 is abnormally acquired. Also according to such a configuration, the first physiological parameter acquired in the acquisition environment low in reliability can be detected.

That is, not only the measurement waveform or the measurement value of the second physiological parameter different from the first physiological parameter of the subject 30 but also the acceleration data or the image data that can represent the body motion of the subject 30 during the acquisition of the first physiological parameter can also be examples of the second data as long as they are data related to the acquisition environment of the first physiological parameter.

In the aforementioned embodiment, both the processing device 11 and the notifier 12 are mounted on the physiological information acquisition device 10. However, the processing device 11 may be mounted on an external server device capable of communicating with the physiological information acquisition device 10 through a communication network. In this case, the processing device 11 executes the aforementioned processing based on the first waveform data W1 and the second waveform data W2 received from the sensor 20 (the sensor 21 if necessary) or the physiological information acquisition device 10 via the communication network. The processing device 11 transmits appropriate control data C through the communication network to control the notifier 12 to execute the notification.

The notifier 12, that executes the notification based on control data C received from the external server device, may be installed in a device that is independent from the physiological information acquisition device 10. For example, the function of the notifier 12 may be implemented by an independent output device that is capable of performing communication with at least one of the physiological information acquisition device 10 and the above external server device via a communication network. In this case, the control data C is transmitted from the output interface 114 of the physiological information acquisition device 10 or the external server device to the output device via the communication network.

## Claims

1. A processing device configured to process physiological information of a subject, the processing device comprising:
a reception interface (111) configured to receive first data (W1) corresponding to a measurement waveform of a first physiological parameter of the subject, and second data (W2) related to an acquisition environment of the first physiological parameter;
a predictor (112) configured to predict a first probability of correctly calculating a value of the first physiological parameter, based on the first data (W1); and
a processor (113) configured to identify, of the first data (W1), a part in which the first probability (P1) is higher than a first threshold (Pt1),
wherein the processor (113) is configured to identify the part as a waveform data part corresponding to an abnormal measurement waveform when the first physiological parameter is determined to be abnormally acquired based on the second data (W2),
wherein the second data corresponds to a measurement waveform of a second physiological parameter of the subject different from the first physiological parameter;
wherein the predictor (112) is configured to predict a second probability of correctly calculating a value of the second physiological parameter, based on the second data; and
wherein the processor (113) is configured to determine that the first physiological parameter is abnormally acquired when the second probability is lower than a second threshold;
wherein the first data is impedance respiration waveform data; and
wherein the second data is electrocardiogram waveform data.

2. The processing device according to Claim 1, wherein:
the reception interface (111) is configured to receive the first data and the second data through a shared sensor.

3. The processing device according to any one of Claims 1 to 2, wherein:
the processor (113) is configured to delete the identified first data part in which the first probability is higher than the first threshold under the circumstance where the first physiological parameter is determined to be abnormally acquired based on the second data.

4. The processing device according to any one of Claims 1 to 2, wherein:
the processor (113) is configured to delete the first data except the identified first data part in which the first probability is higher than the first threshold under the circumstance where the first physiological parameter is determined to be abnormally acquired based on the second data.

5. The processing device according to any one of Claims 1 to 4, wherein:
the processor (113) is configured to update an estimated result about the identified first data part.

6. A computer program that can be executed by a processor of a processing device configured to process physiological information of a subject, wherein when the computer program is executed on the processing device, the processing device performs the steps of:
receiving first data (W1) corresponding to a measurement waveform of a first physiological parameter of the subject, and second data (W2) related to an acquisition environment of the first physiological parameter;
predicting, by means of a predictor (112), a first probability (P1) of correctly calculating a value of the first physiological parameter, based on the first data (W1); and
identifying, by means of the processor (113), of the first data (W1), a part in which the first probability (P1) is higher than a first threshold (Pt1),
wherein the processor (113) is configured to identify the part as a waveform data part corresponding to an abnormal measurement waveform when the first physiological parameter is determined to be abnormally acquired based on the second data (W2), and
wherein the second data corresponds to a measurement waveform of a second physiological parameter of the subject different from the first physiological parameter;
wherein the predictor (112) is configured to predict a second probability of correctly calculating a value of the second physiological parameter, based on the second data; and
wherein the processor (113) is configured to determine that the first physiological parameter is abnormally acquired when the second probability is lower than a second threshold;
wherein the first data is impedance respiration waveform data; and
wherein the second data is electrocardiogram waveform data.

7. The processing device configured to process physiological information of a subject according to any one of claims 1 to 5, comprising:
a notifier (12) configured to send notification about information indicating that the first physiological parameter is abnormally acquired when the first data part identified by the processor is present.

## Patentansprüche

1. Eine Verarbeitungsvorrichtung, die konfiguriert ist, um physiologische Informationen eines Subjekts zu verarbeiten, wobei die Verarbeitungsvorrichtung aufweist:
eine Empfangsschnittstelle (111), die so konfiguriert ist, dass sie erste Daten (W1), die einer Messwellenform eines ersten physiologischen Parameters des Subjekts entsprechen, und zweite Daten (W2), die sich auf eine Erfassungsumgebung des ersten physiologischen Parameters beziehen, empfängt;
einen Prädiktor (112), der so konfiguriert ist, dass er eine erste Wahrscheinlichkeit der korrekten Berechnung eines Wertes des ersten physiologischen Parameters auf der Basis der ersten Daten (W1) vorhersagt; und
einen Prozessor (113), der so konfiguriert ist, dass er von den ersten Daten (W1) einen Teil identifiziert, bei dem die erste Wahrscheinlichkeit (P1) höher ist als ein erster Schwellenwert (Pt1),
wobei der Prozessor (113) so konfiguriert ist, dass er den Teil als einen Wellenformdatenteil identifiziert, der einer abnormalen Messwellenform entspricht, wenn der erste physiologische Parameter auf der Grundlage der zweiten Daten (W2) als abnormal erfasst bestimmt wird,
wobei die zweiten Daten einer Messwellenform eines zweiten physiologischen Parameters des Subjekts entsprechen, der sich von dem ersten physiologischen Parameter unterscheidet;
wobei der Prädiktor (112) so konfiguriert ist, dass er eine zweite Wahrscheinlichkeit der korrekten Berechnung eines Wertes des zweiten physiologischen Parameters auf der Basis der zweiten Daten vorhersagt; und
wobei der Prozessor (113) so konfiguriert ist, dass er feststellt, dass der erste physiologische Parameter abnormal erfasst ist, wenn die zweite Wahrscheinlichkeit niedriger als ein zweiter Schwellenwert ist;
wobei die ersten Daten Impedanzatmungs-Wellenformdaten sind; und
wobei die zweiten Daten Elektrokardiogramm-Wellenformdaten sind.

2. Die Verarbeitungsvorrichtung nach Anspruch 1, wobei:
die Empfangsschnittstelle (111) so konfiguriert ist, dass sie die ersten Daten und die zweiten Daten über einen gemeinsamen Sensor empfängt.

3. Die Verarbeitungsvorrichtung nach einem der Ansprüche 1 bis 2, wobei:
der Prozessor (113) so konfiguriert ist, dass er den identifizierten ersten Datenteil, bei dem die erste Wahrscheinlichkeit höher ist als der erste Schwellenwert, unter dem Umstand löscht, dass der erste physiologische Parameter auf der Grundlage der zweiten Daten als abnormal erfasst bestimmt wird.

4. Die Verarbeitungsvorrichtung nach einem der Ansprüche 1 bis 2, wobei:
der Prozessor (113) so konfiguriert ist, dass er die ersten Daten mit Ausnahme des identifizierten ersten Datenteils löscht, bei dem die erste Wahrscheinlichkeit höher als der erste Schwellenwert ist, und zwar unter dem Umstand, dass der erste physiologische Parameter auf der Grundlage der zweiten Daten als abnormal erfasst bestimmt wird.

5. Die Verarbeitungsvorrichtung nach einem der Ansprüche 1 bis 4, wobei:
der Prozessor (113) konfiguriert ist, um ein geschätztes Ergebnis über den identifizierten ersten Datenteil zu aktualisieren.

6. Ein Computerprogramm, das von einem Prozessor einer Verarbeitungsvorrichtung ausgeführt werden kann, die konfiguriert ist, um physiologische Informationen eines Subjekts zu verarbeiten, wobei, wenn das Computerprogramm auf der Verarbeitungsvorrichtung ausgeführt wird, die Verarbeitungsvorrichtung die folgenden Schritte durchführt:
Empfangen erster Daten (W1), die einer Messwellenform eines ersten physiologischen Parameters des Subjekts entsprechen, und zweiter Daten (W2), die sich auf eine Erfassungsumgebung des ersten physiologischen Parameters beziehen;
Vorhersagen einer ersten Wahrscheinlichkeit (P1) der korrekten Berechnung eines Wertes des ersten physiologischen Parameters mittels eines Prädiktors (112), basierend auf den ersten Daten (W1); und
Identifizieren, mittels des Prozessors (113), der ersten Daten (W1) eines Teils, in dem die erste Wahrscheinlichkeit (P1) höher als ein erster Schwellenwert (Pt1) ist,
wobei der Prozessor (113) konfiguriert ist, um den Teil als einen Wellenformdatenteil zu identifizieren, der einer abnormalen Messwellenform entspricht, wenn der erste physiologische Parameter als abnormal erfasst bestimmt wird, basierend auf den zweiten Daten (W2), und
wobei die zweiten Daten einer Messwellenform eines zweiten physiologischen Parameters des Subjekts entsprechen, der sich von dem ersten physiologischen Parameter unterscheidet;
wobei der Prädiktor (112) so konfiguriert ist, dass er eine zweite Wahrscheinlichkeit der korrekten Berechnung eines Wertes des zweiten physiologischen Parameters auf der Basis der zweiten Daten vorhersagt; und
wobei der Prozessor (113) so konfiguriert ist, dass er bestimmt, dass der erste physiologische Parameter abnormal erfasst ist, wenn die zweite Wahrscheinlichkeit niedriger als ein zweiter Schwellenwert ist;
wobei die ersten Daten Impedanzatmungs-Wellenformdaten sind; und
wobei die zweiten Daten Elektrokardiogramm-Wellenformdaten sind.

7. Die Verarbeitungsvorrichtung, die konfiguriert ist, um physiologische Informationen eines Subjekts gemäß einem der Ansprüche 1 bis 5 zu verarbeiten, aufweisend:
einen Melder (12), der konfiguriert ist, um eine Benachrichtigung über Informationen zu senden, die anzeigen, dass der erste physiologische Parameter abnormal erfasst ist, wenn der erste Datenteil, der durch den Prozessor identifiziert wurde, vorhanden ist.

## Revendications

1. Dispositif de traitement configuré pour traiter des informations physiologiques d'un sujet, le dispositif de traitement comprenant :
une interface de réception (111) configurée pour recevoir de premières données (W1) correspondant à une forme d'onde de mesure d'un premier paramètre physiologique du sujet, et de secondes données (W2) liées à un environnement **d'acquisition** du premier paramètre physiologique ;
un dispositif de prédiction (112) configuré pour prédire une première probabilité de calculer correctement une valeur du premier paramètre physiologique, sur la base des premières données (W1) ; et
un processeur (113) configuré pour identifier, parmi les premières données (W1), une partie dans laquelle la première probabilité (P1) est supérieure à un premier seuil (Pt1),
dans lequel le processeur (113) est configuré pour identifier la partie comme une partie de données de forme d'onde correspondant à une forme d'onde de mesure anormale lorsque le premier paramètre physiologique est déterminé comme étant acquis de manière anormale sur la base des secondes données (W2),
dans lequel les secondes données correspondent à une forme d'onde de mesure d'un second paramètre physiologique du sujet différent du premier paramètre physiologique ;
dans lequel le dispositif de prédiction (112) est configuré pour prédire une seconde probabilité de calculer correctement une valeur du second paramètre physiologique, sur la base des secondes données ; et
dans lequel le processeur (113) est configuré pour déterminer que le premier paramètre physiologique est acquis de manière anormale lorsque la seconde probabilité est inférieure à un second seuil ;
dans lequel les premières données sont des données de forme d'onde de respiration par impédance ; et
dans lequel les secondes données sont des données de forme d'onde d'électrocardiogramme.

2. Dispositif de traitement selon la revendication 1, dans lequel :
l'interface de réception (111) est configurée pour recevoir les premières données et les secondes données à travers un capteur partagé.

3. Dispositif de traitement selon l'une quelconque des revendications 1 à 2, dans lequel :
le processeur (113) est configuré pour effacer la partie des premières données identifiée dans laquelle la première probabilité est supérieure au premier seuil sous la circonstance où le premier paramètre physiologique est déterminé comme étant acquis de manière anormale sur la base des secondes données.

4. Dispositif de traitement selon l'une quelconque des revendications 1 à 2, dans lequel :
le processeur (113) est configuré pour effacer les premières données exceptée la partie des premières données identifiée dans laquelle la première probabilité est supérieure au premier seuil sous la circonstance où le premier paramètre physiologique est déterminé comme étant acquis de manière anormale sur la base des secondes données.

5. Dispositif de traitement selon l'une quelconque des revendications 1 à 4, dans lequel :
le processeur (113) est configuré pour mettre à jour un résultat estimé concernant la partie des premières données identifiée.

6. Programme informatique qui peut être exécuté par un processeur d'un dispositif de traitement configuré pour traiter des informations physiologiques d'un sujet, dans lequel lorsque le programme informatique est exécuté sur le dispositif de traitement, le dispositif de traitement exécute les étapes consistant à :
recevoir de premières données (W1) correspondant à une forme d'onde de mesure d'un premier paramètre physiologique du sujet, et de secondes données (W2) liées à un environnement d'acquisition du premier paramètre physiologique ;
prédire, à l'aide d'un dispositif de prédiction (112), une première probabilité (P1) de calculer correctement une valeur du premier paramètre physiologique, sur la base des premières données (W1) ; et
à identifier, à l'aide du processeur (113), parmi les premières données (W1), une partie dans laquelle la première probabilité (P1) est supérieure à un premier seuil (Pt1),
dans lequel le processeur (113) est configuré pour identifier la partie comme une partie de données de forme d'onde correspondant à une forme d'onde de mesure anormale lorsque le premier paramètre physiologique est déterminé comme étant acquis de manière anormale sur la base des secondes données (W2), et
dans lequel les secondes données correspondent à une forme d'onde de mesure d'un second paramètre physiologique du sujet différent du premier paramètre physiologique ;
dans lequel le dispositif de prédiction (112) est configuré pour prédire une seconde probabilité de calculer correctement une valeur du second paramètre physiologique, sur la base des secondes données ; et
dans lequel le processeur (113) est configuré pour déterminer que le premier paramètre physiologique est acquis de manière anormale lorsque la seconde probabilité est inférieure à un second seuil ;
dans lequel les premières données sont des données de forme d'onde de respiration par impédance ; et
dans lequel les secondes données sont des données de forme d'onde d'électrocardiogramme.

7. Dispositif de traitement configuré pour traiter des informations physiologiques d'un sujet selon l'une quelconque des revendications 1 à 5, comprenant :
un dispositif de notification (12) configuré pour envoyer une notification concernant les informations indiquant que le premier paramètre physiologique est acquis de manière anormale lorsque la partie des premières données identifiée par le processeur est présente.
